# EUROPEAN PATENT APPLICATION

(11) **EP 4 570 246 A1**
(43) Date of publication of application: **18.06.2025**
(21) Application number: 23216952.4
(22) Date of filing: 15.12.2023
(51) Int. Cl.: A61K 31/225, A61K 31/277, A61K 31/433, A61K 31/454, A61K 31/495, A61K 31/502, A61K 31/5025, A61K 31/506, A61K 31/519, A61K 31/7048, A61K 31/713, A61K 38/14, A61K 45/06, A61P 35/00

(54) **COMBINED AGENTS WITH SYNERGISTIC EFFECTS AGAINST GLIOMAS**

(71) Applicant: Eberhard Karls Universität Tübingen Medizinische Fakultät, 72074 Tübingen (DE)
(72) Inventor: Tabatabai, Ghazaleh, 72076 Tübingen (DE); Häußer, Lara Annina, 72076 Tübingen (DE); Trampert, Jil, 72076 Tübingen (DE); Merk, Daniel Josef Wolfgang, 71134 Aidlingen (DE); Kuhlburger, Laurence, 72070 Tübingen (DE)
(74) Representative: Witte, Weller & Partner Patentanwälte mbB

(57) **Abstract**

The present invention relates to a new pharmaceutical composition for the treatment and prophylaxis of a glioma comprising a first active agent combined with at least a second active agent, wherein the combination of said first and said second active agent results in a synergistic effect. The present invention also relates to a first active agent combined with at least a second active agent for use in the treatment and prophylaxis of a glioma wherein the combination of said first and said second active agent results in a synergistic effect. Finally, the invention relates to a method of treatment and prophylaxis of glioma in a subject in need, comprising the administration to the subject of said pharmaceutical composition or the combination of said first and said second active agent.

## Description

The present invention relates to a new pharmaceutical composition for the treatment and prophylaxis of a glioma comprising a first active agent combined with at least a second active agent, wherein the combination of said first and said second active agent results in a synergistic effect. The present invention also relates to a first active agent combined with at least a second active agent for use in the treatment and prophylaxis of a glioma wherein the combination of said first and said second active agent results in a synergistic effect. Finally, the invention relates to a method of treatment and prophylaxis of glioma in a subject in need, comprising the administration to the subject of said pharmaceutical composition or the combination of said first and said second active agent.

### FIELD OF THE INVENTION

The present invention relates to the field of drug development, more particular the field of active agents for the treatment and prophylaxis of a glioma.

Glioblastoma, previously known as glioblastoma multiforme (GBM), is the most aggressive and most common type of cancer that originates in the brain, and has very poor prognosis for survival. The cause of most cases of glioblastoma is not known. Uncommon risk factors include genetic disorders, such as neurofibromatosis and Li-Fraumeni syndrome, and previous radiation therapy. Glioblastomas represent 15% of all brain tumors. They are thought to arise from astrocytes.

Temozolomide (TMZ), sold under the brand name Temodar among others, is an anticancer medication used to treat brain tumors such as glioblastoma and anaplastic astrocytoma. Temozolomide is an imidazotetrazine derivative. The therapeutic benefit of temozolomide depends on its ability to alkylate/methylate DNA, which most often occurs at the N-7 or O-6 positions of guanine residues. This methylation damages the DNA and triggers the death of tumor cells. However, some tumor cells are able to repair this type of DNA damage, and therefore diminish the therapeutic efficacy of temozolomide, by expressing a protein O⁶-alkylguanine DNA alkyltransferase (AGT) encoded in humans by the O-6-methylguanine-DNA methyltransferase (MGMT) gene. In some tumors, epigenetic silencing of the MGMT gene prevents the synthesis of this enzyme, and as a consequence such tumors are more sensitive to killing by temozolomide. For tumors without MGMT promoter methylation TMZ therapy is not as effective as with MGMT promoter methylation. Conversely, the presence of AGT protein in brain tumors predicts poor response to temozolomide and these patients receive little benefit from chemotherapy with temozolomide.

Temozolomide causes a variety of side effects including nausea (feeling sick), vomiting, constipation, loss of appetite, alopecia (hair loss), headache, fatigue (tiredness), convulsions (fits), rash, neutropenia or lymphopenia (low white-blood-cell counts), and thrombocytopenia (low blood platelet counts). People receiving the solution for infusion may also have injection-site reactions, such as pain, irritation, itching, warmth, swelling and redness, as well as bruising.

Nevertheless, the anti-glioblastoma potential of temozolomide is beyond doubt. Due to this potential and due to the availability and establishment of this drug, as well as the high development costs, it is desirable to provide a new and improved use of temozolomide, especially for patients without methylated MGMT promoter. In particular, such a new use should be provided to improve the efficacy of temozolomide in the treatment and prophylaxis of gliomas, especially glioblastomas, and possibly reduce the observed side effects.

Against this background, it is the object of the present invention to find an improved application of temozolomide in the treatment and prophylaxis of gliomas and glioblastomas.

### SUMMARY OF THE INVENTION

This object is met by a pharmaceutical composition for use in the treatment and/or prophylaxis of glioma, said pharmaceutical composition comprising, in pharmaceutically/prophylactically effective amounts, a first active agent combined with at least a second active agent, wherein the combination of said first and said second active agent, when administered to a subject, results in a synergistic effect, wherein said first active agent is temozolomide and said second active agent is an agent inhibiting a target selected from the group consisting of: PARP1, EHMT2, NAE1, CUL3, PIM1, APEX1, GSK3B, LIG1, KEAP1, GSR, and GSTA1.

According to the invention "temozolomide" (Cas no.: 85622-93-1; abbreviated as TMZ; trade names Temodar, Temodal, Temcad, others) is an alkylating agent used to treat serious brain cancers; most commonly as second-line treatments for astrocytoma and as the first-line treatment for glioblastoma. The alkylating cytostatic agent has an antineoplastic effect by disrupting DNA replication in the tumor cell. The substance is a prodrug that is only activated in the body by metabolization. Temozolomide is spontaneously hydrolyzed to form MTIC (= 5-(3-N-methyltriazen-1-yl)-imidazole-4-carboxamide). MTIC is also very unstable and decomposes rapidly in acidic solution into the products AIC (= 5(4)-aminoimidazol-4(5)-carboxamide) and diazomethane, which has a methylating effect after protonation. Diazomethane then methylates bases of the DNA. If this base is located in a promoter region of a gene, the methylation leads to inactivation of the gene, which is then no longer expressed. Temozolomide can cross the blood-brain barrier, which makes it suitable for use in brain tumors.

The inventors used genome-wide CRISPR/Cas9 knockout screens under temozolomide therapy to identify gene losses as functional vulnerabilities. These genetic defects were induced experimentally and a sensitization to temozolomide therapy was detected. Using an established synergy assay, the inventors were able to verify that inhibitors of the identified targets can potentiate the effect of temozolomide. This can reduce the amount of temozolomide used therapeutically or prophylactically and reduce side effects. Especially, the invention can help patients without a methylated MGMT promoter.

"PARP1" or 'Poly [ADP-ribose] polymerase 1' also known as NAD⁺ ADP-ribosyltransferase 1 or poly[ADP-ribose] synthase 1 is an enzyme that in humans is encoded by the PARP1 gene (Entrez: 142, human). It is the most abundant of the PARP family of enzymes, accounting for 90% of the NAD⁺ used by the family. PARP1 is mostly present in cell nucleus, but cytosolic fraction of this protein was also reported. PARP1 acts as a first responder that detects DNA damage and then facilitates choice of repair pathway. PARP1 contributes to repair efficiency by ADP-ribosylation of histones leading to de-compaction of chromatin structure, and by interacting with and modifying multiple DNA repair factors.

"EHMT2" or 'Euchromatic histone-lysine N-methyltransferase 2', also known as G9a, is a histone methyltransferase enzyme that in humans is encoded by the EHMT2 gene (Entrez: 10919, human). G9a catalyzes the mono- and di-methylated states of histone H3 at lysine residue 9 (i.e., H3K9me1 and H3K9me2) and lysine residue 27 (H3K27me1 and HeK27me2). EHMT2 has been shown to interact with KIAA0515 and the prostate tissue associated homeodomain protein NKX3.1.

"NAE1" or 'NEDD8-activating enzyme E1 regulatory subunit' is a protein that in humans is encoded by the NAE1 gene (Entrez: 8883, human). The protein encoded by this gene binds to the beta-amyloid precursor protein. Beta-amyloid precursor protein is a cell surface protein with signal-transducing properties, and it is thought to play a role in the pathogenesis of Alzheimer's disease. In addition, the encoded protein can form a heterodimer with UBE1C and bind and activate NEDD8, a ubiquitin-like protein. This protein is required for cell cycle progression through the S/M checkpoint.

"CUL3" or 'cullin 3' is a protein that in humans is encoded by the CUL3 gene (Entrez: 8452, human). Cullin 3 protein belongs to the family of cullins which in mammals contains eight proteins. Cullin 3 is a component of Cullin-RING E3 ubiquitin ligases complexes (CRLs) which are involved in protein ubiquitylation and represent a part of ubiquitin-proteasome system (UPS). Deregulation of Cullin 3 expression level was observed in human cancers. It was shown that Cullin 3 is overexpressed in invasive cancers, and the protein expression level positively correlates with tumor stage. In breast cancer, the overexpression of Cullin 3 protein results in a decrease of Nrf2 protein level. This protein is a transcription factor regulating the expression of some detoxification and antioxidant enzymes. Another substrate of CRL complex is a candidate tumor suppressor protein RhoBTB2.

"PIM1" or 'Proto-oncogene serine/threonine-protein kinase Pim-1' is an enzyme that in humans is encoded by the PIM1 gene (Entrez: 5292, human). It is a proto-oncogene which encodes for the serine/threonine kinase of the same name. The Pim-1 oncogene was first described in relation to murine T-cell lymphomas, as it was the locus most frequently activated by the Moloney murine leukemia virus. Subsequently, the oncogene has been implicated in multiple human cancers, including prostate cancer, acute myeloid leukemia and other hematopoietic malignancies. Pim-1 is mainly involved in cell cycle progression, apoptosis and transcriptional activation, as well as more general signal transduction pathways.

"APEX1" or 'DNA-(apurinic or apyrimidinic site) lyase' is an enzyme that in humans is encoded by the APEX1 gene (Entrez: 328, human). The APEX gene (alternatively named APE1, HAP1, APEN) encodes the major AP endonuclease in human cells, the central repair proteins addressing apurinic/apyrimidinic (AP) sites (also called "abasic sites"), which are frequently occurring in DNA molecules by spontaneous hydrolysis, by DNA damaging agents or by DNA glycosylases that remove specific abnormal bases. APEX1 has been shown to interact with MUTYH, Flap structure-specific endonuclease 1 and XRCC1.

"GSK3B" or 'Glycogen synthase kinase-3 beta', is an enzyme that in humans is encoded by the GSK3B gene (Entrez: 2932, human). Abnormal regulation and expression of GSK-3 beta is associated with an increased susceptibility towards bipolar disorder. Glycogen synthase kinase-3 (GSK-3) is a proline-directed serine-threonine kinase that was initially identified as a phosphorylating and an inactivating agent of glycogen synthase. Two isoforms, alpha (GSK3A) and beta, show a high degree of amino acid homology. GSK3B is involved in energy metabolism, neuronal cell development, and body pattern formation.

"LIG1" or 'DNA ligase 1' is an enzyme that in humans is encoded by the LIG1 gene (Entrez: 3978, human). DNA ligase I is the only known eukaryotic DNA ligase involved in both DNA replication and repair, making it the most studied of the ligases. It functions in DNA replication and the base excision repair process. DNA ligase 1 is responsible for joining Okazaki fragments formed during discontinuous DNA synthesis on the DNA's lagging strand after DNA polymerase δ has replaced the RNA primer nucleotides with DNA nucleotides. If the Okazaki fragments are not properly ligated together, the unli-gated DNA (containing a 'nick') could easily degrade to a double strand break, a phenomenon known to cause genetic mutations.

"KEAP1" or 'Kelch-like ECH-associated protein 1' is a protein that in humans is encoded by the Keap1 gene (Entrez: 9817, human). KEAP1 has been shown to interact with Nrf2, a master regulator of the antioxidant response, which is important for the amelioration of oxidative stress. Mutations in KEAP1 that result in loss-of-function are not linked to familial cancers, though they do predispose individuals to multinodular goiters. The proposed mechanism leading to goiter formation is that the redox stress experienced when the thyroid produces hormones selects for loss of heterozygosity of KEAP1, leading to the goiters.

"GSR" or 'glutathione-disulfide reductase' is an enzyme that in humans is encoded by the GSR gene (Entrez: 2936, human). GSR catalyzes the reduction of glutathione disulfide (GSSG) to the sulfhydryl form glutathione (GSH), which is a critical molecule in resisting oxidative stress and maintaining the reducing environment of the cell. Glutathione reductase functions as dimeric disulfide oxidoreductase and utilizes an FAD prosthetic group and NADPH to reduce one molar equivalent of GSSG to two molar equivalents of GSH.

"GSTA1" or 'Glutathione S-transferase A1' is an enzyme that in humans is encoded by the GSTA1 gene (Entrez: 2938, human). GSTA1 functions in the detoxification of electrophilic compounds, including carcinogens, therapeutic drugs, environmental toxins and products of oxidative stress, by conjugation with glutathione. Increases in serum and urinary GSTA1 have been found in association with hepatocyte and renal proximal tubular necrosis respectively, and have potential for monitoring injury to these tissues.

"Agents inhibiting said targets" refer to a group of agents which can selectively and specifically target any of the identified targets or genes/gene products, respectively, and reduce or even block its enzymatic activity.

In conclusion, the inventors were able to demonstrate that the combination of the first and at least second active agents results in a synergistic effect with respect to the treatment or prophylaxis of a glioma. "Synergy" according to the invention means that the at least two active agents reinforce each other's effect such that the total effect of the active agents is stronger than a summation (potentiation or superadditive effect).

Methods to determine synergistic effects of at least two active agents are well known in the art, including the Bliss independence model and the Zero interaction potency (ZIP) model; see, e.g., Ma and Motsinger-Reif (2019), Current methods for quantifying drug synergism, Proteom Bioinform. 1(2): 43-48, and Yadav et al. (2015), Searching for drug synergy in complex dose-response landscapes using an interaction potency model, Comput. Struct. Biotechnol. J. 13: 504-513.

According to the invention, "prophylaxis" means any measure intended to prevent a disease or pathologic condition, such as a glioma, from developing or progressing to a pathogenic form.

According to the invention, "treatment" or therapy refers to all measures aimed at positively influencing or improving a disease or pathologic condition, such as a glioma.

According to the invention "therapeutically/prophylactically effective amount" or, synonymously, "effective dose", refer to the amount or dose of the first and/or at least second active agent, that is sufficient to achieve the desired clinical outcome or improvement.

A "subject" according to the invention refers to any living being, including a vertebrate, a mammal, and a human being.

According to the invention the first and/or second active agents also include the respective pharmaceutically acceptable salts thereof, the solvates thereof and the solvates of the salts thereof. Solvates for the purposes of the invention refer to those forms of the active agents, which in the solid or liquid state form a complex by coordination with solvent molecules. Hydrates are a specific form of solvates in which the coordination takes place with water.

In the pharmaceutical composition the first and second active agents can be, in an embodiment of the invention, the only active agents. "At least" a second active agent means that, in alternative embodiments, a third, fourth, fifth etc. active agent is comprised by the pharmaceutical composition according to the invention.

It goes without saying that the pharmaceutical compositing according to the invention may comprise a pharmaceutically acceptable excipient or carrier.

"Pharmaceutically acceptable excipients or carriers" are well known to the skilled person. They allow a proper formulation of the compound according to the invention and serve to improve the selectivity, effectiveness, and/or safety of drug administration. Pharmaceutically acceptable carriers include, without being limited thereto, solvents, fillers, binders, lubricants, stabilizers, surfactants, suspensions, thickeners, emulsifiers, preserving agents, liposomes, micelles, microspheres, nanoparticles, etc. suitable for the particular form of dosage. Except for cases, when the medium of conventional carriers is incompatible with the active ingredient, for example, upon occurrence of any undesirable biological effects or other adverse interactions with any other ingredient(s) of the pharmaceutical composition, the use of such compositions falls within the scope of this invention. Materials that can serve as pharmaceutically acceptable carriers include, but are not limited to, monosaccharides and oligosaccharides, as well as derivatives thereof; malt, gelatin; talc; excipients such as: cocoa butter and suppository waxes; oils, such as peanut oil, cotton-seed oil, safflower oil, sesame oil, olive oil, corn oil and soybean oil; glycols such as propylene glycol; esters, such as ethyl oleate and ethyl laurate; agar; buffering agents, such as magnesium hydroxide and aluminum hydroxide; alginic acid; pyrogen-free water; isotonic saline, Ringer's solution; ethyl alcohol and phosphate buffer solutions. In addition, the composition may contain other non-toxic compatible lubricants, for example sodium lauryl sulfate and magnesium stearate, as well as coloring agents, parting liquids, film formers, sweeteners, flavoring additives and flavorants, preserving agents and antioxidants.

The phrase "pharmaceutically acceptable" refers to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

Suitable pharmaceutical acceptable carriers or excipients as well as pharmaceutical necessities for use in pharmaceutical formulations are described in Remington - The Science and Practice of Pharmacy, 23rd edition, 2020, a well-known reference text in this field, and in the USP/NF (United States Pharmacopeia and the National Formulary). Other sources are also available to one of ordinary skill in the art.

"Comprising" according to the invention generally means encompassing all the specifically mentioned features as well as optional, additional unspecified ones. However, according to embodiments of the invention, "comprising" also includes "consisting of" which means that only those features are included which are explicitly specified.

The problem underlying the invention is hereby completely solved.

According to an embodiment of the invention said second agent is selected from the group consisting of: dimethylfumarate, bardoxolone methyl, olaparib, EHMT2-IN-1, vitexin, pevonedistat, tideglusib, bleomycin A2, AZD1208, NBDHEX, SGI-1776, and nilotinib.

This measure has the advantage that existing, well-defined substances are used which, according to the inventors' findings, are particularly suitable as second agents.

"Dimethylfumarate" (CAS no.: 624-49-7), also known as fumaric acid dimethyl ester, is the diester of fumaric acid with the alcohol methanol. This and other esters of fumaric acid are inserted as drugs for the treatment of multiple sclerosis and psoriasis. Dimethylfumarate is said to have immunomodulatory and antioxidant properties.

"Bardoxolone methyl" (CAS no.: 218600-53-4) is an experimental and orally-bioavailable semi-synthetic triterpenoid, based on the scaffold of the natural product oleanolic acid. Pre-clinical studies indicate that the compound acts as an activator of the Nrf2 pathway and an inhibitor of the NF-κB pathway.

"Olaparib" (CAS no.: 763113-22-0), sold under the brand name Lynparza, is a medication for the maintenance treatment of BRCA-mutated advanced ovarian cancer in adults. It is a PARP inhibitor, inhibiting poly ADP ribose polymerase (PARP), an enzyme involved in DNA repair. It acts against cancers in people with hereditary BRCA1 or BRCA2 mutations, which include some ovarian, breast, and prostate cancers.

"EHMT2-IN-1" (CAS. no.: 2230849-55-3) is a potent EHMT inhibitor, with IC₅₀s of all <100 nM for EHMT1 peptide, EHMT2 peptide and cellular EHMT2. It is currently used in the research of blood disorder or cancer.

"Vitexin" (CAS no.: 3681-93-4) is a naturally occurring chemical compound. As 8-C-glucosyl-apigenin, vitexin is a derivative of apigenin, a plant pigment from the flavonoid group. Strictly speaking, the C-glycoside is not a true glycoside, but is often assigned to this group. Like many other flavonoids, vitexin has an antioxidant effect.

"Pevonedistat" (MLN4924) (CAS no.: 905579-51-3) is a selective NEDD8 inhibitor. Pevonedistat is an AMP mimetic. Pevonedistat forms a stable covalent adduct with NEDD8, which cannot be utilized in subsequent reactions necessary for the activity of so-called NEDD8-activating enzyme (NAE). Inhibition of NAE prevents activation of cullin-RING ligases (CRLs), which are critical for proteasome-mediated protein degradation. Pevonedistat has been shown to disrupt CRL-mediated protein turnover leading to apoptosis in cancer cells by deregulating S-phase DNA synthesis. Essentially, it encourages apoptosis in dividing cells.

"Tideglusib" (NP-12, NP031112) (CAS no.: 865854-05-3) is a potent and irreversible small molecule glycogen synthase kinase 3 (GSK-3) inhibitor. The use of tideglusib specifically was associated with mild-moderate adverse reactions, which included transient increases in serum creatine kinase, ALT-or gGT-diarrhea, nausea, cough, fatigue, and headache. Like other GSK3 inhibitors tideglusib is discussed in the context of the treatment of neurological disorders. In a phase-Ila clinical trial for Alzheimer's disease, the treatment was discontinued, due to lack of efficacy.

"Bleomycin" (CAS nos.:11056-06-7, 9041-93-4 *-sulfate,* 67763-87-5 *-hydrochloride*) is a cytostatic drug that is used in the treatment of cancer (chemotherapy) and belongs to the so-called tumor antibiotics. Bleomycin acts by induction of DNA strand breaks. Some studies suggest bleomycin also inhibits incorporation of thymidine into DNA strands. DNA cleavage by bleomycin depends on oxygen and metal ions, at least *in vitro.* The exact mechanism of DNA strand scission is unresolved, but it has been suggested that bleomycin chelates metal ions (primarily iron), producing a pseudoenzyme that reacts with oxygen to produce superoxide and hydroxide free radicals that cleave DNA. The drug bleomycin is produced by fermentation in bacterial cultures and obtained from the culture filtrate by ion exchange absorption. It is essentially composed of two individual compounds of the bleomycin substance group: 55 - 70 % of the dimethylsulfonium aminopropyl derivative (= bleomycin A2) and 25 - 32 % of the agmatine derivative (= bleomycin B2) of bleomycin acid; the total content of bleomycin A2+B2 is at least 85 %.

"AZD1208" (CAS no.: 1204144-28-4) is an orally bioavailable, highly selective PIM kinases inhibitor. AZD1208 induces autophagy, cell cycle arrest and apoptosis.

"NBDHEX" (CAS no.: 787634-60-0) is a potent glutathione S-transferase P1-1 (GSTP1-1) inhibitor. NBDHEX induces apoptosis of tumor cells. NBDHEX acts as an anticancer agent by inhibiting GSTs catalytic activity, avoiding inconvenience of the inhibitor extrusion from the cell by specific pumps and disrupting the interaction between the GSTP1-1 and key signaling effectors. NBDHEX can also act as late-phase autophagy inhibitor.

"SGI-1776" (CAS no.: 1025065-69-3) is an ATP competitive inhibitor of Pim1 with IC₅₀ of 7 nM in a cell-free assay, 50- and 10-fold selective versus Pim2 and Pim3, also potent to Flt3 and haspin. SGI-1776 induces apoptosis and autophagy.

"Nilotinib" (trade name: Tasigna^{®}, manufacturer: Novartis) (CAS no.: 641571-10-0) is a protein kinase inhibitor that was developed under the name AMN107. It is a specific BCR-ABL tyrosine kinase inhibitor and works by interfering with signaling within the cancer cell.

In still another embodiment said second agent is acting on the target via RNA interference (RNAi).

This measure has the advantage that inhibitors are used that can be customized against any of the identified targets. RNA interference (RNAi) is a biological process in which RNA molecules are involved in sequence-specific suppression of gene expression by double-stranded RNA, through translational or transcriptional repression. Synthetic double-stranded RNA (dsRNA) introduced into cells can selectively and robustly induce suppression of specific genes of interest.

In another embodiment of the invention said second agent is selected from the group consisting of: short hairpin RNA (shRNA), microRNA (miRNA), and small interfering RNA (siRNA).

This measure is advantageous in that highly suitable molecules are already being employed that make use of the RNA interference (RNAi) mechanism.

A short hairpin RNA or small hairpin RNA (shRNA/hairpin vector) is an artificial RNA molecule with a tight hairpin turn that can be used to silence target gene expression via RNAi. It typically consists of a stem of 19-29 base pairs (bp), a loop of at least 4 nucleotides (nt), and a dinucleotide overhang at the 3' end. Expression of shRNA in cells is typically accomplished by delivery of plasmids or through viral or bacterial vectors. shRNA is an advantageous mediator of RNAi in that it has a relatively low rate of degradation and turnover.

Small interfering RNA (siRNA), sometimes known as short interfering RNA or silencing RNA, is a class of double-stranded RNA at first non-coding RNA molecules, typically 20-24 (normally 21) base pairs in length, similar to miRNA, and operating within the RNAi pathway. It interferes with the expression of specific genes with complementary nucleotide sequences by degrading mRNA after transcription, preventing translation.

MicroRNA (miRNA) are small, single-stranded, non-coding RNA molecules containing 21 to 23 nucleotides. Mature miRNAs are structurally similar to siRNAs produced from exogenous dsRNA, but before reaching maturity, miRNAs must first undergo extensive post-transcriptional modification.

Each of the first and/or second active agent, in another embodiment, is present per dosage unit of the pharmaceutical composition at a concentration that, upon administration into a subject, results in a concentration in the glioma cells which is approx. ≤ 2000 µM, preferably about ≤ 1000 µM, further preferably ≤ 500 µM, further preferably ≤ 200 µM, further preferably ≤ 150 µM, further preferably ≤ 100 µM, further preferably ≤ 50 µM, further preferably ≤ 25 µM, further preferably ≤ 20 µM, further preferably ≤ 10 µM, further preferably ≤ 5 µM, further preferably ≤ 4 µM, further preferably ≤ 3 µM, further preferably ≤ 2.5 µM, further preferably ≤ 2 µM, and further preferably ≤ 1 µM.

This measure takes into account the findings of the inventors that setting the right therapeutic window by using the specified concentrations ensures on the one hand that the anti-glioma activity of the active agents is sufficient and on the other hand any cytotoxic effects are minimized.

In still another embodiment of the invention the glioma is a glioblastoma.

Glioblastoma, previously known as glioblastoma multiforme (GBM), is the most aggressive and most common type of cancer that originates in the brain, and has very poor prognosis for survival. There is no known method of preventing this cancer. Treatment usually involves surgery, after which chemotherapy and radiation therapy are used. However, the results achieved are often unsatisfactory and usually short-lived. Therefore, the invention provides a remedy here for the first time and provides an improved therapy and prophylaxis option.

In another embodiment of the invention the synergistic effect is determinable by the Bliss independence model and the Zero interaction potency (ZIP) model.

This measure has the advantage that the models are used which were al-so used by the inventors to find the combinations of active agents. They are therefore particularly suitable for determining the synergy. Both methods are well known to the skilled person; see, e.g., Ma and Motsinger-Reif (*loc*. *cit.*) and Yadav et al. (*loc. cit.*).

Another subject-matter of the invention relates to a first active agent combined with at least a second active agent for use in the treatment and/or prophylaxis of a glioma, wherein the combination of said first and said second active agent, when administered to a subject, results in a synergistic therapeutic and/or prophylactic effect, wherein said first active agent is temozolomide and said second active agent is an agent inhibiting a target selected from the group consisting of: PARP1, EHMT2, NAE1, CUL3, PIM1, APEX1, GSK3B, LIG1, KEAP1, GSR, and GSTA1.

The embodiments, properties, advantages, and features of the pharmaceutical composition according to the invention apply in a corresponding way to the combination for use according to the invention.

A still further subject-matter of the invention relates to a method of treatment and/or prophylaxis of glioma in a subject in need, comprising the administration to said subject of the pharmaceutical composition according to the invention or the first active agent combined with at least a second active agent for use according to the invention.

The embodiments, properties, advantages, and features of the pharmaceutical composition according to the invention apply in a corresponding way to the method according to the invention.

In an embodiment of the method according to the invention the first and the second active agent are administered simultaneously. In another embodiment of the method according to the invention the first and the second active agent are administered separately.

Due to this method of administration, their respective benefits can be selectively exploited and external circumstances of treatment can be accommodated. Simultaneous administration facilitates administration and promotes patient compliance or adherence to therapy. Separate administration can induce a priming effect via the agent administered first.

It is to be understood that the before-mentioned features and those to be mentioned in the following cannot only be used in the combination indicated in the respective case, but also in other combinations or in an isolated manner without departing from the scope of the invention.

The invention is now further explained by means of embodiments and examples resulting in additional features, characteristics and advantages of the invention. The embodiments are of pure illustrative nature and do not limit the scope or range of the invention. The features mentioned in the specific embodiments are general features of the invention which are not only applicable in the specific embodiment but also in an isolated manner and in the context of any embodiment of the invention.

### BRIEF DESCRIPTION OF THE FIGURES

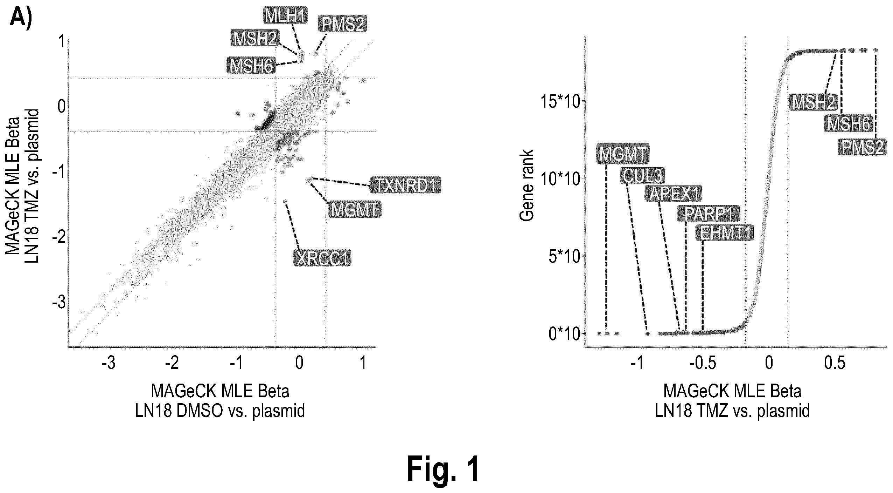

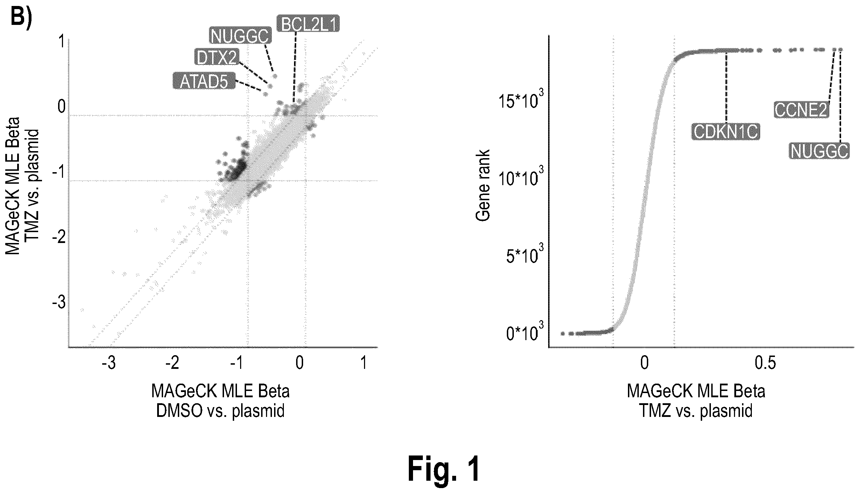

- Fig. 1: CRISPR/Cas9 screen analyses using an activation library (B) in GS-9, LN18, LN229, LNZ308 and T98G cells and knockout library (A) in MGMT promoter methylated/AGT expressing cell lines LN18 and T98G to identify modifiers of response to TMZ. Left: Scatter plot of MAGeCK MLE results from comparing Calabrese (B) or Brunello (A) sgRNA distributions from DMSO or TMZ-treated cells to the plasmid library pool merged for all cell lines. Right: Rankview plot illustrating MAGeCK MLE results from comparing Calabrese (B) or Brunello (A) sgRNA distributions from TMZ-treated cells to the corresponding DMSO control merged for all cell lines.
- Fig. 2: Acute cytotoxicity synergy assay results in LN18 cells of combination treatments of TMZ with dimethylfumarate (A), olaparib (B), EHMT2-IN-1 (C), vitexin (D), pevonedistat (E), and tideglusib (F).
- Fig. 3: Heatmap with all synergy scores of drug combinations with TMZ in four glioma cell lines LN18, T98G, LN229, and LNZ308. Data normalized within each cell line. On the left are the drug targets listed which were identified in the CRISPR/Cas9 screens.
- Fig. 4: Acute cytotoxicity assay of glioblastoma primary cultures treated with TMZ in combination with pevonedistat (A), EHMT2-IN-1 (B), SGI-1776 (C), nilotinib (D), and NBDHEX (E) for 72 h.

### EXAMPLES

### 1. Material and methods

### Acute cytotoxicity assay

The inventors seeded either 5,000 or 10,000 cells, treated them on the following day, and incubated them for 72 h. Following this incubation, cell viability was assessed using CellTiterBlue reagent (Promega, Madison, Wl, US) in accordance with the manufacturer's instructions, utilizing a GloMax (Promega, Madison, Wl, US).

### Synergy assay

To assay drug combinations, the inventors treated the cells in 4x4 matrixes with temozolomide and a second drug following the acute cytotoxicity assay protocol. The inventors analyzed the data for synergistic interactions with the R package synergyfinder to calculate zero interaction potential (ZIP) synergy scores for each tested combination in the 4x4 matrix and the average across the plate. Mean averages of two independent runs for each combination were then normalized over all combinations tested within a cell line. The highest average synergy value was set as 100% and a synergy value of 0 was set as 0%.

### Clonogenic survival assay

The inventors seeded the cells one day prior to treatment. The inventors then treated the cells with the specified concentrations in serum-free medium for 24 hours. Afterward, they changed the medium back to one containing FCS and incubated the cells for a period ranging from 14 to 21 days. The inventors stained the colonies using 0.5% w/v crystal violet and counted them. Measurements were normalized to the respective vehicle control wells.

### Primary glioblastoma cultures

The inventors used fresh residual material obtained from primary tumor tissue after resection at the Department of Neurosurgery, University Hospital Tübingen, to obtain primary glioblastoma cultures (approved by the ethics committee of the University Hospital Tübingen, vote number 225/2023 BO2). The inventors cut the tissue into small pieces, washed with Hanks Balanced Salt Solution (HBSS) (Gibco, Carlsbad, CA, US), and then digested using collagenase and dispase (Roche, Basel, CH). They used red blood cell lysis buffer (Sigma Aldrich, St. Louis, MO, US) to remove the remaining erythrocytes. For treatment, the inventors followed the acute cytotoxicity assay protocol. They treated the cells with temozolomide and a second drug in monotherapy and combination. The inventors then used the Bliss Independence Criterion to evaluate synergism potential. They calculated a predicted value for additivity, the product of the two monotherapies, and compared it with the observed measurement of the combination treatment. Lower observed values compared to the prediction indicate towards synergy, higher towards antagonism.

### 2. Results

To identify mechanisms involved in temozolomide (TMZ) treatment in glioblastoma, the inventors performed genome-wide CRISPR/Cas9 activation and knockout screens under treatment with TMZ or DMSO control. Activation screens (Figure 1 b) were performed in five glioma cell lines GS-9, LN229, LN18, LNZ308, and T98G using the Calabrese sgRNA library. Sequencing data was analyzed using MAGeCK MLE. Genes enriched in the TMZ treated cells but not in the DMSO treated cells might confer resistance against treatment. Here the inventors identified genes which can be targeted with drugs. For the genome-wide CRISPR/Cas9 knockout screens the inventors used Brunello sgRNA library in two O-6-methylguanine-DNA methyltransferase (MGMT) promoter unmethylated or protein expressing glioma cell lines LN18 and T98G (Figure 1 a). For tumors without MGMT promoter methylation a high need for new treatment option exists because TMZ monotherapy which is part of the standard of care treatment is not as effective as with MGMT promoter methylation. In the knockout screens the enriched genes include members of the DNA mismatch repair like MSH2, MLH1, PMS2, which are necessary for the effect of TMZ on cells, so loss of these genes leads to cell survival. Depleted genes in the TMZ treated sample which were not affected in the DMSO treated sample are synthetic lethal interactors of the treatment. Among those depleted genes the inventors identified PARP1, EHMT2, NAE1, CUL3, PIM1, APEX1, GSK3B, LIG1, KEAP1, GSR, and GSTA1 which can be targeted with different drugs.

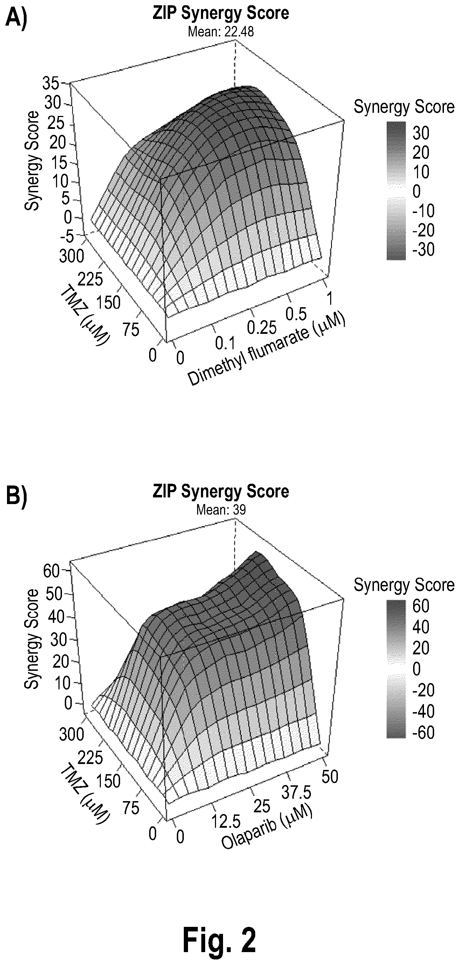

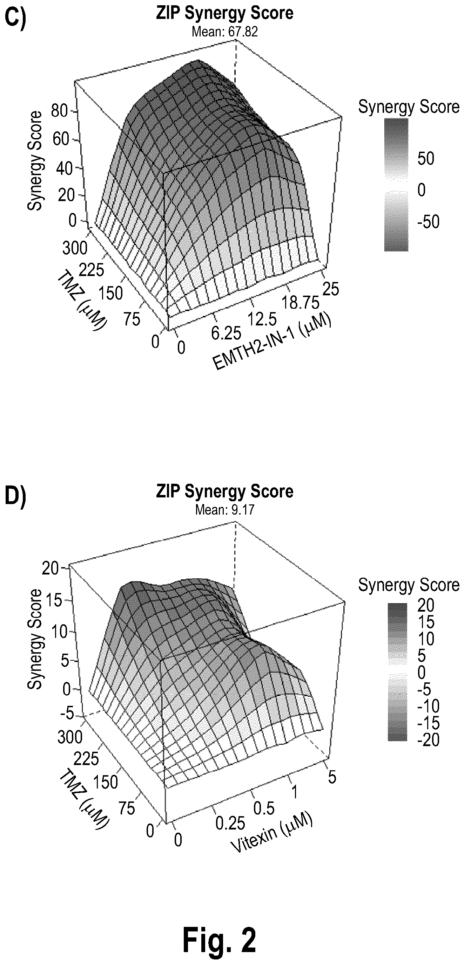

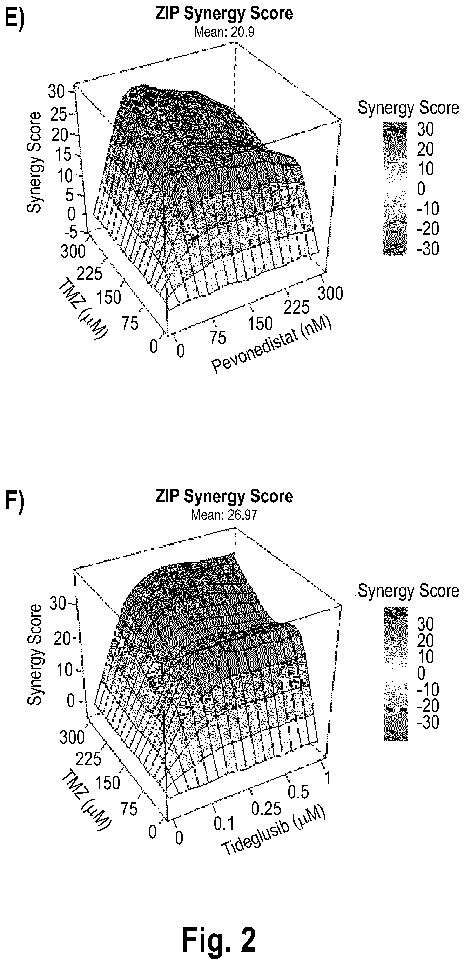

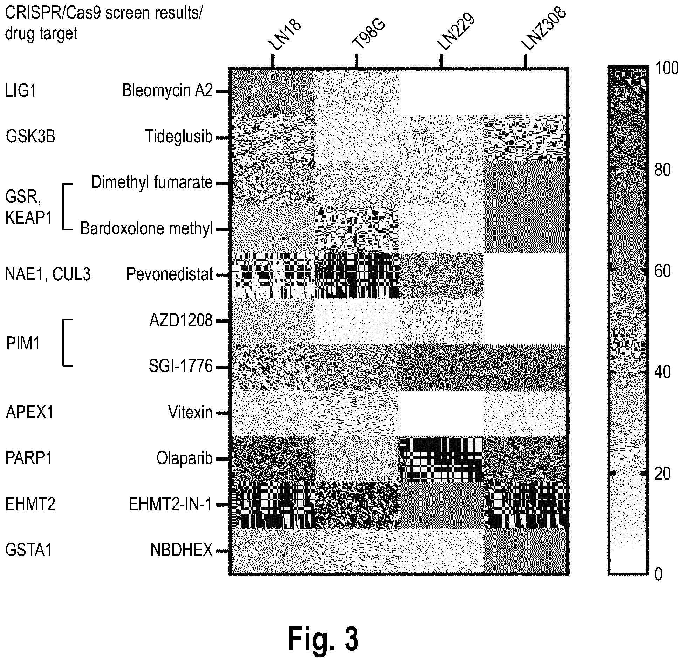

The combined inhibition of synthetic lethal interactors leads to cell death. Therefore, drugs targeting the different identified gene products were used in acute cytotoxicity synergy assays in combination with TMZ to analyze the synergistic effect of drug combinations. The inventors tested 11 drugs in 72-hour acute cytotoxicity synergy assay in combination with TMZ on six glioma cell lines (three without MGMT promoter methylation/protein expression, three with MGMT promoter methylation/without protein expression) in two independent runs. In Figure 2 the combinations of TMZ with dimethylfumarate, Olaparib, EHMT2-IN-1, vitexin, pevonedistat, and tideglusib are shown in one representative run in LN18 cell line (MGMT methylated). The calculated ZIP synergy score for each combination indicates if a combination is synergistic, the higher the score the stronger is the synergism. For the heatmap shown in Figure 3 (includes only four of the tested cell lines; LN18 and T98G MGMT promoter methylated/protein expressing, LN229 and LNZ308 MGMT promoter unmethylated/without protein expression) the synergy scores were normalized within each cell line taking the highest synergy score detected in this cell line as 100% and a synergy score of 0 as 0%. For each target shown here identified in the CRISPR/Cas9 screens, we detected a synergistic effect of the drug combination.

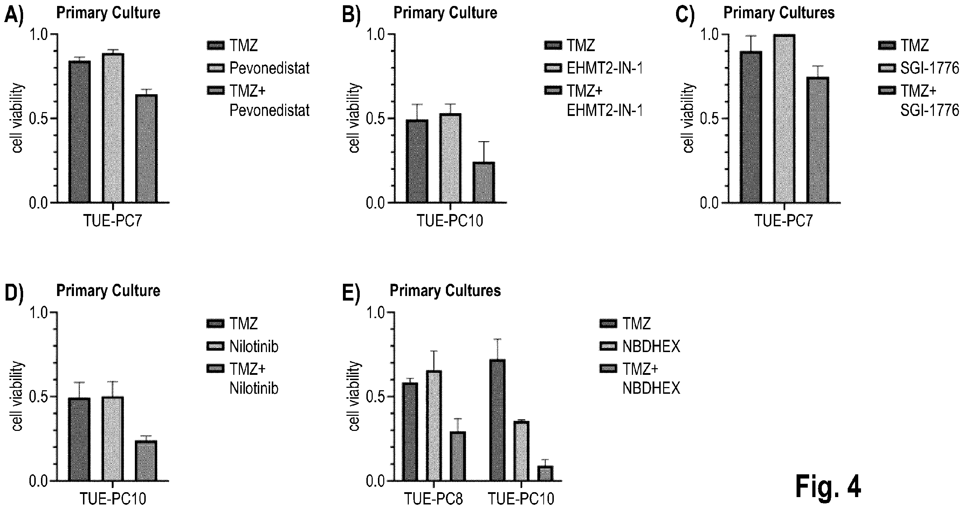

The inventors extracted primary cells from glioblastoma tissue to test the drug combinations *ex vivo.* Analyzing the combination effect on cell viability of TMZ with five different drugs in glioblastoma primary cultures (Figure 4), the observed effect of the combination treatment was in each combination synergistic compared to the predicted effect calculated from both monotherapies (Bliss synergy score, data not shown). Here the inventors additionally included the combination of TMZ with nilotinib which targets amongst others KIT which was enriched in the CRISPR/Cas9 activation screens when looking only at MGMT unmethylated cell lines.

### 3. Conclusion

With the present invention, the inventors provide for the first time a combination of active ingredients comprising temozolomide and a second active agent inhibiting a specific target which, due to the observed clear synergistic effects of both components in the treatment and prophylaxis of gliomas, can be expected to lead to a significant therapeutic improvement over current treatment concepts. The inventors were able to demonstrate these effects in established experimental systems using representative example combinations.

## Claims

1. A pharmaceutical composition for use in the treatment and/or prophylaxis of glioma, said pharmaceutical composition comprising, in pharmaceutically/prophylactically effective amounts, a first active agent combined with at least a second active agent, wherein the combination of said first and said second active agent, when administered to a subject, results in a synergistic effect, wherein said first active agent is temozolomide and said second active agent is an agent inhibiting a target selected from the group consisting of: PARP1, EHMT2, NAE1, CUL3, PIM1, APEX1, GSK3B, LIG1, KEAP1, GSR, and GSTA1.

2. The pharmaceutical composition for use of claim 1, wherein said second agent is selected from the group consisting of: dimethylfumarate, bardoxolone methyl, olaparib, EHMT2-IN-1, vitexin, pevonedistat, tideglusib, bleomycin A2, AZD1208, NBDHEX, SGI-1776, and nilotinib.

3. The pharmaceutical composition for use of claim 1 or 2, wherein said second agent is acting on the target via RNA interference (RNAi).

4. The pharmaceutical composition for use of any of the preceding claims, wherein said second agent is selected from the group consisting of: short hairpin RNA (shRNA), microRNA (miRNA), and small interfering RNA (siRNA).

5. The pharmaceutical composition of any of the preceding claims, wherein the glioma is a glioblastoma.

6. The pharmaceutical composition of any of the preceding claims, wherein the synergistic effect is determinable by Bliss independence model and the Zero interaction potency (ZIP) model.

7. A first active agent combined with at least a second active agent for use in the treatment and/or prophylaxis of a glioma, wherein the combination of said first and said second active agent, when administered to a subject, results in a synergistic therapeutic and/or prophylactic effect, wherein said first active agent is temozolomide and said second active agent is an agent inhibiting a target selected from the group consisting of: PARP1, EHMT2, NAE1, CUL3, PIM1, APEX1, GSK3B, LIG1, KEAP1, GSR, and GSTA1.

8. The first active agent combined with at least a second active agent for use of claim 7, wherein said second agent is selected from the group consisting of: dimethylfumarate, bardoxolone methyl, olaparib, EHMT2-IN-1, vitexin, pevonedistat, tideglusib, bleomycin A2, AZD1208, NBDHEX, SGI-1776, and nilotinib.

9. The first active agent combined with at least a second active agent for use of claim 7 or 8, wherein said second agent is acting on the target via RNA interference (RNAi).

10. The first active agent combined with at least a second active agent for use of claim 9, wherein said second agent is selected from the group consisting of: short hairpin RNA (shRNA), microRNA (miRNA), and small interfering RNA (siRNA).

11. A method of treatment and/or prophylaxis of glioma in a subject in need, comprising the administration to said subject of the pharmaceutical composition of any of claims 1-6 or the first active agent combined with at least a second active agent for use of any of claims 7-10.
